# EUROPEAN PATENT SPECIFICATION

(11) **EP 1 238 986 B1**
(45) Date of publication and mention of the grant of the patent: **25.06.2008**
(21) Application number: 02006351.7
(22) Date of filing: 28.10.1992
(51) Int. Cl.: C07K 16/28, A61K 39/395, C07K 14/715, A61K 38/17

(54) **Use of Vascular endothelial cell growth factor antagonists**
Verwendung von Antagonisten des Zellwachstumsfaktors VEGF
Utilisation d'antagonistes du facteur de croissance cellulaire VEGF

(43) Date of publication of application: 11.09.2002
(62) Divisional of application: 92923512.5
(73) Proprietor: GENENTECH, INC., South San Francisco, CA 94080-4990 (US)
(72) Inventor: Ferrara, Napoleone, San Francisco, CA 94109 (US); Kim, Kyung Jin, San Francisco, CA 94112 (US)
(74) Representative: Denison, Christopher Marcus

(56) References cited:
- WO-A-92/14748
- US-A- 5 036 003
- TERMAN B I ET AL: "IDENTIFICATION OF THE KDR TYROSINE KINASE AS A RECEPTOR FOR VASCULAR ENDOTHELIAL CELL GROWTH FACTOR" BIOCHEMICAL AND BIOPHYSICAL RESEARCH COMMUNICATIONS, ACADEMIC PRESS INC. ORLANDO, FL, US, vol. 187, no. 3, 30 September 1992 (1992-09-30), pages 1579-1586, XP002013861 ISSN: 0006-291X
- FOLKMAN J ET AL: "ANGIOGENIC FACTORS" SCIENCE, AMERICAN ASSOCIATION FOR THE ADVANCEMENT OF SCIENCE,, US, vol. 235, 23 January 1987 (1987-01-23), pages 442-447, XP002065073 ISSN: 0036-8075
- UENO H ET AL: "INHIBITION OF PDGF BETA RECEPTOR SIGNAL TRANSDUCTION BY COEXPRESSION OF A TRUNCATED RECEPTOR" SCIENCE, AMERICAN ASSOCIATION FOR THE ADVANCEMENT OF SCIENCE,, US, vol. 252, 10 May 1991 (1991-05-10), pages 844-848, XP002056525 ISSN: 0036-8075
- KIM K J ET AL: "THE VASCULAR ENDOTHELIAL GROWTH FACTOR PROTEINS: IDENTIFICATION OF BIOLOGICALLY RELEVANT REGIONS BY NEUTRALIZING MONOCLONAL ANTIBODIES" GROWTH FACTORS, HARWOOD ACADEMIC PUBLISHERS GMBH, XX, vol. 7, no. 1, December 1992 (1992-12), pages 53-64, XP000612291 ISSN: 0897-7194

## Description

### Field of the Invention

The present invention relates to vascular endothelial cell growth factor (VEGF) antagonists, to therapeutic compositions comprising the antagonists, and to methods of use of the antagonists for diagnostic and therapeutic purposes.

### Background of the Invention

The two major cellular components of the vasculature are the endothelial and smooth muscle cells. The endothelial cells form the lining of the inner surface of all blood vessels, and constitute a nonthrombogenic interface between blood and tissue. In addition, endothelial cells are an important component for the development of new capillaries and blood vessels. Thus, endothelial cells proliferate during the angiogenesis, or neovascularization, associated with tumor growth and metastasis, and a variety of non-neoplastic diseases or disorders.

Various naturally occurring polypeptides reportedly induce the proliferation of endothelial cells. Among those polypeptides are the basic and acidic fibroblast growth factors (FGF), Burgess and Maciag, Annual Rev. Biochem., 58:575 (1989), platelet-derives endothelial cell growth factor (PD-ECGF), Ishikawa, et al., Nature, 338:557 (1989), and vascular endothelial growth factor (VEGF), Leung, et al., Science 246:1306 (1989); Ferrara & Henzel, Biochem. Biophys. Res. Commun. 161:851 (1989); Tischer, et al., Biochem. Biophys. Res. Commun. 165:1198 (1989); Ferrara, et al., PCT Pat. Pub. No. WO 90/13649 (published November 15, 1990); Ferrara, et al., U.S. Pat. App. No. 07/360,229.

VEGF was first identified in media conditioned by bovine pituitary follicular or folliculostellate cells. Biochemical analyses indicate that bovine VEGF is a dimeric protein with an apparent molecular mass of approximately 45,000 Daltons, and with an apparent mitogenic specificity for vascular endothelial cells. DNA encoding bovine VEGF was isolated by screening a cDNA library prepared from such cells, using oligonucleotides based on the amino-terminal amino acid sequence of the protein as hybridization probes.

Human VEGF was obtained by first screening a cDNA library prepared from human cells, using bovine VEGF cDNA as a hybridization probe. One cDNA identified thereby encodes a 165-amino acid protein having greater than 95% homology to bovine VEGF, which protein is referred to as human VEGF (hVEGF). The mitogenic activity of human VEGF was confirmed by expressing the human VEGF cDNA in mammalian host cells. Media conditioned by cells transfected with the human VEGF cDNA promoted the proliferation of capillary endothelial cells, whereas control cells did not. Leung, et al., Science 246:1306 (1989).

Several additional cDNAs were identified in human cDNA libraries that encode 121-, 189-, and 206-amino acid isoforms of hVEGF (also collectively referred to as hVEGF-related proteins). The 121-amino acid protein differs from hVEGF by virtue of the deletion of the 44 amino acids between residues 116 and 159 in hVEGF. The 189-amino acid protein differs from hVEGF by virtue of the insertion of 24 amino acids at residue 116 in hVEGF, and apparently is identical to human vascular permeability factor (hVPF). The 206-amino acid protein differs from hVEGF by virtue of an insertion of 41 amino acids at residue 116 in hVEGF. Houck, et al., Mol. Endocrin. 5:1806 (1991); Ferrara, et al., J. Cell. Biochem. 47:211 (1991); Ferrara, et al., Endocrine Reviews 13:18 (1992); Keck, et al., Science 246:1309 (1989); Connolly, et al., J. Biol. Chem. 264:20017 (1989); Keck, et al., EPO Pat. Pub. No. 0 370 989 (published May 30, 1990).

VEGF not only stimulates vascular endothelial cell proliferation, but also induces vascular permeability and angiogenesis. Angiogenesis, which involves the formation of new blood vessels from preexisting endothelium, is an important component of a variety of diseases and disorders including tumor growth and metastasis, rheumatoid arthritis, psoriasis, atherosclerosis, diabetic retinopathy, retrolental fibroplasia, neovascular glaucoma, hemangiomas, immune rejection of transplanted corneal tissue and other tissues, and chronic inflammation.

In the case of tumor growth, angiogenesis appears to be crucial for the transition from hyperplasia to neoplasia, and for providing nourishment to the growing solid tumor. Folkman, et al., Nature 339:58 (1989). Angiogenesis also allows tumors to be in contact with the vascular bed of the host, which may provide a route for metastasis of the tumor cells. Evidence for the role of angiogenesis in tumor metastasis is provided, for example, by studies showing a correlation between the number and density of microvessels in histologic sections of invasive human breast carcinoma and actual presence of distant metastases. Weidner, et al., New Engl. J. Med. 324:1 (1991).

In view of the role of vascular endothelial cell growth and angiogenesis, and the role of those processes in many diseases and disorders, it is desirable to have a means of reducing or inhibiting one or more of the biological effects of VEGF. It is also desirable to have a means of assaying for the presence of VEGF in normal and pathological conditions, and especially cancer.

### Summary of the Invention

The present invention as defined in the claims provides the use of antagonists of VEGF, including (a) antibodies and variants thereof which are capable of specifically binding to hVEGF or hVEGF receptor and (b) hVEGF receptor and variants thereof in the manufacture of a medicament for the treatment of non-neoplastic diseases or disorders characterized by undesirable excessive neovascularization, including by way of example trheumatoid arthritis, psoriasis, atherosclerosis, diabetic and other retinopathies, retrolental fibroplasia, neovascular glaucoma, hemangiomas, thyroid hyperplasias (including Grave's disease), corneal and other tissue transplantation, and chronic inflammation.

The VEGF antagonist may be polyspecific monoclonal antibodies which are capable of binding to (a) a non-hVEGF epitope, for example, an epitope of a protein involved in thrombogenesis or thrombolysis, or a tumor cell surface antigen, and to (b) hVEGF or hVEGF receptor.

The VEGF antagonists may be conjugated with a cytotoxic moiety.

The VEGF antagonist may be for coadministration, either simultaneously or sequentially, with one or more other VEGF antagonists or anti-angiogenic substances.

### Brief Description of the Drawings

Figure 1 shows the effect of anti-hVEGF monoclonal antibodies (A4.6.1 or B2.6.2) or an irrelevant anti-hepatocyte growth factor antibody (anti-HGF) on the binding of the anti-hVEGF monoclonal antibodies to hVEGF.
Figure 2 shows the effect of anti-hVEGF monoclonal antibodies (A4.6.1 or B2.6.2) or an irrelevant anti-HGF antibody on the biological activity of hVEGF in cultures of bovine adrenal cortex capillary endothelial (ACE) cells.
Figure 3 shows the effect of anti-hVEGF monoclonal antibodies (A4.6.1, B2.6.2, or A2.6.1) on the binding of hVEGF to bovine ACE cells.
Figure 4 shows the effect of A4.6.1 anti-hVEGF monoclonal antibody treatment on the rate of growth of growth of NEG55 tumors in mice.
Figure 5 shows the effect of A4.6.1 anti-hVEGF monoclonal antibody treatment on the size of NEG55 tumors in mice after five weeks of treatment.
Figure 6 shows the effect of A4.6.1 anti-hVEGF monoclonal antibody (VEGF Ab) treatment on the growth of SK-LMS-1 tumors in mice.
Figure 7 shows the effect of varying doses of A4.6.1 anti-hVEGF monoclonal antibody (VEGF Ab) treatment on the growth of A673 tumors in mice. is shown in
Figure 8 shows the effect of A4.6.1 anti-hVEGF monoclonal antibody on the growth and survival of NEG55 (G55) glioblastoma cells in culture.
Figure 9 shows the effect of A4.6.1 anti-hVEGF monoclonal antibody on the growth and survival of A673 rhabdomyosarcoma cells in culture.
Figure 10 shows the effect of A4.6.1 anti-hVEGF monoclonal antibody on human synovial fluid-induced chemotaxis of human endothelial cells.

### Detailed Description of the Invention

The term "hVEGF" as used herein refers to the 165-amino acid human vascular endothelial cell growth factor, and related 121-, 189-, and 206-amino acid vascular endothelial cell growth factors, as described by Leung, et al., Science 246:1306 (1989), and Houck, et al., Mol. Endocrin. 5:1806 (1991) together with the naturally occurring allelic and processed forms of those growth factors.

The present invention provides antagonists of hVEGF which are capable of inhibiting one or more of the biological activities of hVEGF, for example, its mitogenic or angiogenic activity. Antagonists of hVEGF act by interfering with the binding of hVEGF to a cellular receptor.Included within the scope of the invention are antibodies, and preferably monoclonal antibodies, or fragments thereof, that bind to hVEGF or hVEGF receptor. Also included within the scope of the invention are hVEGF receptor and fragments and amino acid sequence variants thereof which are capable of binding hVEGF.

The term "hVEGF receptor" or "hVEGFr" as used herein refers to a cellular for hVEGF, ordinarily a cell-surface receptor found on vascular endothelial cells, as well as variants thereof which retain the ability to bind hVEGF. Typically, the hVEGF receptors and variants thereof that are hVEGF antagonists will be in isolated form, rather than being integrated into a cell membrane or fixed to a cell surface as may be the case in nature. One example of a hVEGF receptor is the fms-like tyrosine kinase (flt), a transmembrane receptor in the tyrosine kinase family. DeVries, et al., Science 255:989 (1992); Shibuya, et al., Oncogene 5:519 (1990). The flt receptor comprises an extracellular domain, a transmembrane domain, and an intracellular domain with tyrosine kinase activity. The extracellular domain is involved in the binding of hVEGF, whereas the intracellular domain is involved in signal transduction.

Another example of an hVEGF receptor is the flk-1 receptor (also referred to as KDR). Matthews, et al., Proc. Nat. Acad. Sci. 88:9026 (1991); Terman, et al., Oncogene 6:1677 (1991); Terman, et al., Biochem. Biophys. Res. Commun. 187:1579 (1992).

Binding of hVEGF to the flt receptor results in the formation of at least two high molecular weight complexes, having apparent molecular weight of 205,000 and 300,000 Daltons. The 300,000 Dalton complex is believed to be a dimer comprising two receptor molecules bound to a single molecule of hVEGF.

Variants of hVEGFr also are included within the scope hereof. Representative examples include truncated forms of a receptor in which the transmembrane and cytoplasmic domains are deleted from the receptor, and fusions proteins in which non-hVEGFr polymers or polypeptides are conjugated to the hVEGFr or, preferably, truncated forms thereof. An example of such a non-hVEGF polypeptide is an immunoglobulin. In that case, for example, the extracellular domain of the hVEGFr is substituted for the Fv domain of an immunoglobulin light or (preferably) heavy chain, with the C-terminus of the receptor extracellular domain covalently joined to the amino terminus of the CH1, hinge, CH2 or other fragment of the heavy chain. Such variants are made in the same fashion as known immunoadhesons. See e.g., Gascoigne, et al., Proc. Nat. Acad. Sci. 84:2936 (1987); Capon, et al., Nature 337:525 (1989); Aruffo, et al., Cell 61:1303 (1990); Ashkenazi, et al., Proc. Nat. Acad. Sci. 88:10535 (1991); Bennett, et al., J. Biol. Chem. 266:23060 (1991). In other embodiments, the hVEGFr is conjugated to a non-proteinaceous polymer such as polyethylene glycol (PEG) (see e.g., Davis, et al., U.S. Patent No. 4,179,337; Goodson, et al., BioTechnology 8:343-346 (1990); Abuchowski, et al., J. Biol. Chem. 252:3578 (1977); Abuchowski, et al., J. Biol. Chem. 252:3582 (1977)) or carbohydrates (see e.g., Marshall, et al., Arch. Biochem. Biophys., 167:77 (1975)). This serves to extend the biological half-life of the hVEGFr and reduces the possibility that the receptor will be immunogenic in the mammal to which it is administered. The hVEGFr is used in substantially the same fashion as antibodies to hVEGF, taking into account the affinity of the antagonist and its valency for hVEGF.

The extracellular domain of hVEGF receptor, either by itself or fused to an immunoglobulin polypeptide or other carrier polypeptide, is especially useful as an antagonist of hVEGF, by virtue of its ability to sequester hVEGF that is present in a host but that is not bound to hVEGFr on a cell surface.

hVEGFr and variants thereof also are useful in screening assays to identify agonists and antagonists of hVEGF. For example, host cells transfected with DNA encoding hVEGFr (for example, fit or flk1) overexpress the receptor polypeptide on the cell surface, making such recombinant host cells ideally suited for analyzing the ability of a test compound (for example, a small molecule, linear or cyclic peptide, or polypeptide) to bind to hVEGFr. hVEGFr and hVEGFr fusion proteins, such as an hVEGFr-IgG fusion protein, may be used in a similar fashion. For example, the fusion protein is bound to an immobilized support and the ability of a test compound to displace radiolabeled hVEGF from the hVEGFr domain of the fusion protein is determined.

The term "recombinant" used in reference to hVEGF, hVEGF receptor, monoclonal antibodies, or other proteins, refers to proteins that are produced by recombinant DNA expression in a host cell. The host cell may be prokaryotic (for example, a bacterial cell such as E. coli) or eukaryotic (for example, a yeast or a mammalian cell).

### Antagonist Monoclonal Antibodies

The term "monoclonal antibody" as used herein refers to an antibody obtained from a population of substantially homogeneous antibodies, i.e., the individual antibodies comprising the population are identical in specificity and affinity except for possible naturally occurring mutations that may be present in minor amounts. It should be appreciated that as a result of such naturally occurring mutations and the like, a monoclonal antibody composition of the invention, which will predominantly contain antibodies capable of specifically binding hVEGF or hVEGFr may also contain minor amounts of other antibodies.

Thus, the modifier "monoclonal" indicates the character of the antibody as being obtained from such a substantially homogeneous population of antibodies, and is not to be construed as requiring production of the antibody by any particular method. For example, monoclonal antibodies of the invention may be made using the hybridoma method first described by Kohler & Milstein, Nature 256:495 (1975), or may be made by recombinant DNA methods. Cabilly, et al., U.S. Pat. No. 4,816,567.

In the hybridoma method, a mouse or other appropriate host animal is immunized with antigen by subcutaneous, intraperitoneal, or intramuscular routes to elicit lymphocytes that produce or are capable of producing antibodies that will specifically bind to the protein(s) used for immunization. Alternatively, Iymphocytes may be immunized in vitro. Lymphocytes then are fused with myeloma cells using a suitable fusing agent, such as polyethylene glycol, to form a hybridoma cell. Goding, Monoclonal Antibodies: Principles and Practice, pp.59-103 (Academic Press, 1986).

The antigen may be hVEGF or hVEGFr. The antigen optionally is a fragment or portion of any one of hVEGF or hVEGFr having one or more amino acid residues that participate in the binding of hVEGF to one of its receptors. For example, immunization with the extracellular domain of an hVEGFr (that is, a truncated hVEGFr polypeptide lacking transmembrane and intracellular domains) will be especially useful in producing antibodies that are antagonists of hVEGF, since it is the extracellular domain that is involved in hVEGF binding.

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high level expression of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, SP-2 cells available from the American Type Culture Collection, Rockville, Maryland USA, and P3X63Ag8U.1 cells described by Yelton, et al., Curr. Top. Microbiol. Immunol. 81:1 (1978). Human myeloma and mouse-human heteromyeloma cell lines also have been described for the production of human monoclonal antibodies. Kozbor, J. Immunol. 133:3001 (1984). Brodeur, et al., Monoclonal Antibody Production Techniques and Applications, pp.51-63 (Marcel Dekker, Inc., New York, 1987).

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA). The monoclonal antibodies of the invention are those that preferentially immunoprecipitate hVEGF or hVEGFr or that preferentially bind to at least one of those antigens in a binding assay, and that are capable of inhibiting a biological activity of hVEGF.

After hybridoma cells are identified that produce antagonist antibodies of the desired specificity, affinity, and activity, the clones may be subcloned by limiting dilution procedures and grown by standard methods. Goding, Monoclonal Antibodies: Principles and Practice, pp.59-104 (Academic Press, 1986). Suitable culture media for this purpose include, for example, Dulbecco's Modified Eagle's Medium or RPMI-1640 medium. In addition, the hybridoma cells may be grown in vivo as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

DNA encoding the monoclonal antibodies of the invention is readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese Hamster ovary (CHO) cells, or myeloma cells that do not otherwise produce immunoglobulin protein, to obtain the synthesis of monoclonal antibodies in the recombinant host cells.

The DNA optionally may be modified in order to change the character of the immunoglobulin produced by its expression. For example, humanized forms of murine antibodies are produced by substituting a complementarity determining region (CDR) of the murine antibody variable domain for the corresponding region of a human antibody. In some embodiments, selected framework region (FR) amino acid residues of the murine antibody also are substituted for the corresponding amino acid residues in the human antibody. Carter, et al., Proc. Nat. Acad. Sci. 89:4285 (1992); Carter, et al., BioTechnology 10:163 (1992). Chimeric forms of murine antibodies also are produced by substituting the coding sequence for selected human heavy and light constant chain domains in place of the homologous murine sequences. Cabilly, et al., U.S. Pat. No. 4,816,567; Morrison, et al., Proc. Nat. Acad. Sci. 81:6851 (1984).

The antibodies included within the scope of the invention include variant antibodies, such as chimeric (including "humanized") antibodies and hybrid antibodies comprising immunoglobulin chains capable of binding hVEGF or hVEGFr, and a non-hVEGF epitope.

The antibodies herein include all species of origin, and immunoglobulin classes (e.g., IgA, IgD, IgE, IgG, and IgM) and subclasses, as well as antibody fragments (e.g., Fab, F(ab')₂, and Fv), so long as they are capable of binding hVEGFor hVEGFr. and are capable of antagonizing a biological activity of hVEGF.

In a preferred embodiment of the invention, the monoclonal antibody will have an affinity for the immunizing antigen of at least about 10⁹ liters/mole, as determined, for example, by the Scatchard analysis of Munson & Pollard, Anal. Biochem. 107:220 (1980). Also, the monoclonal antibody typically will inhibit the mitogenic or angiogenic activity of hVEGF at least about 50%, preferably greater than 80%, and most preferably greater than 90%, as determined, for example, by an in vitro cell survival or proliferation assay, such as described in Example 2.

For some therapeutic and diagnostic applications, it is desirable that the monoclonal antibody be reactive with fewer than all of the different molecular forms of hVEGF. For example, it may be desirable to have a monoclonal antibody that is capable of specifically binding to the 165-amino acid sequence hVEGF but not to the 121- or 189-amino acid sequence hVEGF polypeptides. Such antibodies are readily identified by comparative ELISA assays or comparative immunoprecipitation of the different hVEGF polypeptides.

### Conjugates with Cytotoxic Moieties

In some embodiments it is desireable to provide a cytotoxic moiety conjugated to a hVEGF-specific monoclonal antibody or to hVEGFr. In these embodiments the cytotoxin serves to incapacitate or kill cells which are expressing or binding hVEGF or its receptor. The conjugate is targeted to the cell by the domain which is capable of binding to hVEGF or hVEGFr. Thus, monoclonal antibodies that are capable of binding hVEGF or hVEGFr are conjugated to cytotoxins. Similarly, hVEGFr is conjugated to a cytotoxin. While the monoclonal antibodies optimally are capable of neutralizing the activity of hVEGF alone (without the cytotoxin), it is not necessary in this embodiment that the monoclonal antibody or receptor be capable of any more than binding to hVEGF or hVEGFr.

Typically, the cytotoxin is a protein cytotoxin, e.g. diptheria, ricin or Pseudomonas toxin, although in the case of certain classes of immunoglobulins the Fc domain of the monoclonal antibody itself may serve to provide the cytotoxin (e.g., in the case of lgG2 antibodies, which are capable of fixing complement and participating in antibody-dependent cellular cytotoxicity (ADCC)). However, the cytotoxin does not need to be proteinaceous and may include chemotherapeutic agents heretofore employed, for example, for the treatment of tumors.

The cytotoxin typically is linked to a monoclonal antibody or fragment thereof by a backbone amide bond within (or in place of part or all of) the Fc domain of the antibody. Where the targeting function is supplied by hVEGFr, the cytotoxic moiety is substituted onto any domain of the receptor that does not participate in hVEGF binding; preferably, the moiety is substituted in place of or onto the transmembrane and or cytoplasmic domains of the receptor. The optimal site of substitution will be determined by routine experimentation and is well within the ordinary skill.

Conjugates which are protein fusions are easily made in recombinant cell culture by expressing a gene encoding the conjugate. Alternatively, the conjugates are made by covalently crosslinking the cytotoxic moiety to an amino acid residue side chain or C-terminal carboxyl of the antibody or the receptor, using methods known per se such as disulfide exchange or linkage through a thioester bond using for example iminothiolate and methyl-4-mercaptobutyrimadate.

### Conjugates with other Moieties

The monoclonal antibodies and hVEGFr that are antagonists of hVEGF also are conjugated to substances that may not be readily classified as cytotoxins in their own right, but which augment the activity of the compositions herein. For example, monoclonal antibodies or hVEGFr capable of binding to hVEGF or hVEGFr are fused with heterologous polypeptides, such as viral sequences, with cellular receptors, with cytokines such as TNF, interferons, or interleukins, with polypeptides having procoagulant activity, and with other biologically or immunologically active polypeptides. Such fusions are readily made by recombinant methods. Typically such non-immunoglobulin polypeptides are substituted for the constant domain(s) of an anti-hVEGF antibody, or for the transmembrane and/or intracellular domain of an hVEGFr. Alternatively, they are substituted for a variable domain of one antigen binding site of an anti-hVEGF antibody described herein.

In preferred embodiments, such non-immunoglobulin polypeptides are joined to or substituted for the constant domains of an antibody described herein. Bennett, et al., J. Biol. Chem. 266:23060-23067 (1991). Alternatively, they are substituted for the Fv of an antibody herein to create a chimeric polyvalent antibody comprising at least one remaining antigen binding site having specificity for hVEGF or hVEGFr, and a surrogate antigen binding site having a function or specificity distinct from that of the starting antibody.

### Heterospecific Antibodies

Monoclonal antibodies capable of binding to hVEGF or hVEGFr need only contain a single binding site for the enumerated epitopes, typically a single heavy-light chain complex or fragment thereof. However, such antibodies optionally also bear antigen binding domains that are capable of binding an epitope not found within any one of hVEGF or hVEGFr. For example, substituting the corresponding amino acid sequence or amino acid residues of a native anti-hVEGF or anti-HVEGFr antibody with the complementarity-determing and, if necessary, framework residues of an antibody having specificity for an antigen other than hVEGF or hVEGFr will create a polyspecific antibody comprising one antigen binding site having specificity for hVEGF or hVEGFr and another antigen binding site having specificity for the non-hVEGF or hVEGFr antigen. These antibodies are at least bivalent, but may be polyvalent, depending upon the number of antigen binding sites possessed by the antibody class chosen. For example, antibodies of the IgM class will be polyvalent.

In preferred embodiments of the invention such antibodies are capable of binding an hVEGF or hVEGFr epitope and either (a) a polypeptide active in blood coagulation, such as protein C or tissue factor, (b) a cytotoxic protein such as tumor necrosis factor (TNF), or (c) a non-hVEGFr cell surface receptor, such as CD4, or HER-2 receptor (Maddon, et al., Cell 42:93 (1985); Coussens, et al., Science 230:1137 (1985)). Heterospecific, multivalent antibodies are conveniently made by cotransforming a host cell with DNA encoding the heavy and light chains of both antibodies and thereafter recovering, by immunoaffinity chromatography or the like, the proportion of expressed antibodies having the desired antigen binding properties. Alternatively, such antibodies are made by in vitro recombination of monospecific antibodies.

### Monovalent Antibodies

Monovalent antibodies capable of binding to hVEGFr are especially useful as antagonists of hVEGF. Without limiting the invention to any particular mechanism of biological activity, it is believed that activation of cellular hVEGF receptors proceeds by a mechanism wherein the binding of hVEGF to cellular hVEGF receptors induces aggregation of the receptors, and in turn activates intracellular receptor kinase activity. Because monovalent anti-hVEGF receptor antibodies cannot induce such aggregation, and therefore cannot activate hVEGF receptor by that mechanism, they are ideal antagonists of hVEGF.

It should be noted, however, that these antibodies should be directed against the hVEGF binding site of the receptor or should otherwise be capable of interfering with hVEGF binding to the receptor hVEGF, such as by sterically hindering hVEGF access to the receptor. As described elsewhere herein, however, anti-hVEGFr antibodies that are not capable of interfering with hVEGF binding are useful when conjugated to non-immunoglobulin moieties, for example, cytotoxins.

Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking. In vitro methods are also suitable for preparing monovalent antibodies. For example, Fab fragments are prepared by enzymatic cleavage of intact antibody.

### Diagnostic Uses

For diagnostic applications, the antibodies or hVEGFr of the invention typically will be labeled with a detectable moiety. The detectable moiety can be any one which is capable of producing, either directly or indirectly, a detectable signal. For example, the detectable moiety may be a radioisotope, such as ³H, ¹⁴C, ³²P, ³⁶S, or ¹²⁵I, a fluorescent or chemiluminescent compound, such as fluorescein isothiocyanate, rhodamine, or luciferin; radioactive isotopic labels, such as, e.g., ¹²⁶I, ³²P, ¹⁴C, or ³H, or an enzyme, such as alkaline phosphatase, beta-galactosidase or horseradish peroxidase.

Any method known in the art for separately conjugating the antibody or hVEGFr to the detectable moiety may be employed, including those methods described by Hunter, et al., Nature 144:945 (1962); David, et al., Biochemistry 13:1014 (1974); Pain, et al., J. Immunol. Meth. 40:219 (1981); and Nygren, J. Histochem. and Cytochem. 30:407 (1982).

The antibodies and receptors of the present invention may be employed in any known assay method, such as competitive binding assays, direct and indirect sandwich assays, and immunoprecipitation assays. Zola, Monoclonal Antibodies: A Manual of Techniques, pp.147-158 (CRC Press, Inc., 1987).

Competitive binding assays rely on the ability of a labeled standard (which may be hVEGF or an immunologically reactive portion thereof) to compete with the test sample analyte (hVEGF) for binding with a limited amount of antibody. The amount of hVEGF in the test sample is inversely proportional to the amount of standard that becomes bound to the antibodies or receptors. To facilitate determining the amount of standard that becomes bound, the antibodies or receptors generally are insolubilized before or after the competition, so that the standard and analyte that are bound to the antibodies or receptors may conveniently be separated from the standard and analyte which remain unbound.

Sandwich assays involve the use of two antibodies or receptors, each capable of binding to a different immunogenic portion, or epitope, of the protein to be detected. In a sandwich assay, the test sample analyte is bound by a first antibody or receptor which is immobilized on a solid support, and thereafter a second antibody binds to the analyte, thus forming an insoluble three part complex. David & Greene, U.S. Pat No. 4,376,110. The second antibody or receptor may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

The antibodies or receptor herein also is useful for in vivo imaging, wherein an antibody or hVEGFr labeled with a detectable moiety is administered to a patient, preferably into the bloodstream, and the presence and location of the labeled antibody or receptor in the patient is assayed. This imaging technique is useful, for example, in the staging and treatment of neoplasms. The antibody or hVEGFr is labeled with any moiety that is detectable in a mammal, whether by nuclear magnetic resonance, radiology, or other detection means known in the art.

### Antagonist Variants of hVEGF

In addition to the antibodies described herein, other useful antagonists of hVEGF include fragments and amino acid sequence variants of native hVEGF that bind to hVEGF receptor but that do not exhibit the biological activity of native hVEGF. For example, such antagonists include fragments and amino acid sequence variants that comprise a receptor binding domain of hVEGF, but that lack a domain conferring biological activity, or that otherwise are defective in activating cellular hVEGF receptors, such as in the case of a fragment or an amino acid sequence variant that is deficient in its ability to induce aggregation or activation of cellular hVEGF receptors. The term "receptor binding domain" refers to the amino acid sequences in hVEGF that are involved in hVEGF receptor binding. The term "biological activity domain" or "domain conferring biological activity" refers to an amino acid sequence in hVEGF that confer a particular biological activity of the factor, such as mitogenic or angiogenic activity.

The observation that hVEGF appears to be capable of forming a complex with two or more hVEGFr molecules on the surface of a cell suggests that hVEGF has at least two discrete sites for binding to hVEGFr and that it binds to such cellular receptors in sequential fashion, first at one site and then at the other before activation occurs, in the fashion of growth hormone, prolactin and the like (see e.g., Cunningham, et al., Science 254:821 (1991); deVos, et al., Science 255:306 (1992); Fuh, et al., Science 256:1677 (1992)). Accordingly, antagonist variants of hVEGF are selected in which one receptor binding site of hVEGF (typically the site involved in the initial binding of hVEGF to hVEGFr) remains unmodified (or if modified is varied to enhance binding), while a second receptor binding site of hVEGF typically is modified by nonconservative amino acid residue substitution(s) or deletion(s) in order to render that binding site inoperative.

Receptor binding domains in hVEGF and hVEGF binding domains in hVEGFr are determined by methods known in the art, including X-ray studies, mutational analyses, and antibody binding studies. The mutational approaches include the techniques of random saturation mutagenesis coupled with selection of escape mutants, and insertional mutagenesis. Another strategy suitable for identifying receptor-binding domains in ligands is known as alanine (Ala)-scanning mutagenesis. Cunningham, et al., Science 244, 1081-1985 (1989). This method involves the identification of regions that contain charged amino acid side chains. The charged residues in each region identified (i.e. Arg, Asp, His, Lys, and Glu) are replaced (one region per mutant molecule) with Ala and the receptor binding of the obtained ligands is tested, to assess the importance of the particular region in receptor binding. A further powerful method for the localization of receptor binding domains is through the use of neutralizing anti-hVEGF antibodies. Kim, et al., Growth Factors 7:53 (1992). Usually a combination of these and similar methods is used for localizing the domains involved in receptor binding.

The term "amino acid sequence variant" used in reference to hVEGF refers to polypeptides having amino acid sequences that differ to some extent from the amino acid sequences of the native forms of hVEGF. Ordinarily, antagonist amino acid sequence variants will possess at least about 70% homology with at least one receptor binding domain of a native hVEGF, and preferably, they will be at least about 80%, more preferably at least about 90% homologous with a receptor binding domain of a native hVEGF. The amino acid sequence variants possess substitutions, deletions, and/or insertions at certain positions within the amino acid sequence of native hVEGF, such that the variants retain the ability to bind to hVEGF receptor (and thus compete with native hVEGF for binding to hVEGF receptor) but fail to induce one or more of the biological effects of hVEGF, such as endothelial cell proliferation, angiogenesis, or vascular permeability.

"Homology" is defined as the percentage of residues in the amino acid sequence variant that are identical with the residues in the amino acid sequence of a receptor binding domain of a native hVEGF after aligning the sequences and introducing gaps, if necessary, to achieve the maximum percent homology. Methods and computer programs for the alignment are well known in the art. One such computer program is "Align 2", authorec by Genentech, Inc., which was filed with user documentation in the United States Copyright Office, Washington, DC 20559, on December 10, 1991. Substitutional variants are those that have at least one amino acid residue in a native sequence removed and a different amino acid inserted in its place at the same position. The substitutions may be single, where only one amino acid in the molecule has been substituted, or they may be multiple, where two or more amino acids have been substituted in the same molecule.

Insertional variants are those with one or more amino acids inserted immediately adjacent to an amino acid at a particular position in a native sequence. Immediately adjacent to an amino acid means connected to either the α-carboxy or α-amino functional group of the amino acid.

Deletional variants are those with one or more amino acid residues in a native sequence removed. Ordinarily, deletional variants will have one or two amino acid residues deleted in a particular region of the molecule.

Fragments and amino acid sequence variants of hVEGF are readily prepared by methods known in the art, such as by site directed mutagenesis of the DNA encoding the native factor. The mutated DNA is inserted into an appropriate expression vector, and host cells are then transfected with the recombinant vector. The recombinant host cells and grown in suitable culture medium, and the desired fragment or amino acid sequence variant expressed in the host cells then is recovered from the recombinant cell culture by chromatographic or other purification methods.

Alternatively, fragments and amino acid variants of hVEGF are prepared in vitro, for example by proteolysis of native hVEGF, or by synthesis using standard solid-phase peptide synthesis procedures as described by Merrifield (J. Am. Chem. Soc. 85:2149 (1963]), although other equivalent chemical syntheses known in the art may be used. Solid-phase synthesis is initiated from the C-terminus of the peptide by coupling a protected α-amino acid to a suitable resin. The amino acids are coupled to the peptide chain using techniques well known in the art for the formation of peptide bonds.

### Therapeutic Uses

For therapeutic applications, the antagonists of the invention are administered to a mammal, preferably a human, in a pharmaceutically acceptable dosage form, including those that may be administered to a human intravenously as a bolus or by continuous infusion over a period of time, by intramuscular, intraperitoneal, intra-cerobrospinal, subcutaneous, intra-articular, intrasynovial, intrathecal, oral, topical, or inhalation routes. The antagonists also are suitably administered by intralesional, or perilesional routes, to exert local as well as systemic therapeutic effects.

Such dosage forms encompass pharmaceutically acceptable carriers that are inherently nontoxic and nontherapeutic. Examples of such carriers include ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts, or electrolytes such as protamine sulfate, disodium hydrogen phosphate, potassium hydrogen phosphate, sodium chloride, zinc salts, colloidal silica, magnesium trisilicate, polyvinyl pyrrolidone, cellulose-based substances, and polyethylene glycol. Carriers for topical or gel-based forms of antagonist include polysaccharides such as sodium carboxymethylcellulose or methylcellulose, polyvinylpyrrolidone, polyacrylates, polyoxyethylene-polyoxypropylene-block polymers, polyethylene glycol, and wood wax alcohols. For all administrations, conventional depot forms are suitably used. Such forms include, for example, microcapsules, nano-capsules, liposomes, plasters, inhalation forms, nose sprays, sublingual tablets, and sustained-release preparations. The antagonist will typically be formulated in such vehicles at a concentration of about 0.1 mg/ml to 100 mg/ml.

Suitable examples of sustained release preparations include semipermeable matrices of solid hydrophobic polymers containing the antagonist, which matrices are in the form of shaped articles, e.g. films, or microcapsules. Examples of sustained-release matrices include polyesters, hydrogels (for example, poly(2-hydroxyethyl-methacrylate) as described by Langer et al., J. Biomed. Mater. Res. 15:167 (1981) and Langer, Chem. Tech., 12: 98-105 (1982), or poly(vinylalcohol), polylactides (U.S. Pat. No. 3,773,919), copolymers of L-glutamic acid and gamma ethyl-L-glutamate (Sidman et al., Biopolymers, 22:547 (1983), non-degradable ethylene-vinyl acetate (Langer et al., supra), degradable lactic acid-glycolic acid copolymers such as the Lupron Depot™ (injectable micropheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated polypeptide antagonists remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

Sustained-release hVEGF antagonist compositions also include liposomally entrapped antagonist antibodies and hVEGFr. Liposomes containing the antagonists are prepared by methods known in the art, such as described in Epstein, et al., Proc. Natl. Acad. Sci. USA, 82:3688 (1985); Hwang, et al., Proc. Natl. Acad. Sci. USA, 77:4030 (1980); U.S. Patent No. 4,485,045; U.S. Patent No. 4,544,545. Ordinarily the liposomes are the small (about 200-800 Angstroms) unilamelar type in which the lipid content is greater than about 30 mol.% cholesterol, the selected proportion being adjusted for the optimal HRG therapy. Liposomes with enhanced circulation time are disclosed in U.S. Patent No. 5,013,556.

Another use of the present invention comprises incorporating an hVEGF antagonist into formed articles. Such articles can be used in modulating endothelial cell growth and angiogenesis. In addition, tumor invasion and metastasis may be modulated with these articles.

For the prevention or treatment of disease, the appropriate dosage of antagonist will depend on the type of disease to be treated, as defined above, the severity and course of the disease, whether the antibodies are administered for preventive or therapeutic purposes, previous therapy, the patient's clinical history and response to the antagonist, and the discretion of the attending physician. The antagonist is suitably administered to the patient at one time or over a series of treatments.

The hVEGF antagonists are useful in the treatment of various non-neoplastic diseases and disorders.

Non-neoplastic conditions that are amenable to treatment include rheumatoid arthritis, psoriasis, atherosclerosis, diabetic and other retinopathies, retrolental fibroplasia, neovascular glaucoma, thyroid hyperplasias (including Grave's disease), corneal and other tissue transplantation, chronic inflammation, lung inflammation, nephrotic syndrome, preeclampsia, ascites, pericardial effusion (such as that associated with pericarditis), and pleural effusion.

Depending on the type and severity of the disease, about 1 µg/kg to 15 mg/kg of antagonist is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is repeated until a desired suppression of disease symptoms occurs. However, other dosage regimens may be useful. The progress of this therapy is easily monitored by conventional techniques and assays.

According to another embodiment of the invention, the effectiveness of the antagonist in preventing or treating disease may be improved by administering the antagonist serially or in combination with another agent that is effective for those purposes, such as tumor necrosis factor (TNF), an antibody capable of inhibiting or neutralizing the angiogenic activity of acidic or basic fibroblast growth factor (FGF) or hepatocyte growth factor (HGF), an antibody capable of inhibiting or neutralizing the coagulant activities of tissue factor, protein C, or protein S (see Esmon, et al., PCT Patent Publication No. WO 91/01753, published 21 February 1991), or one or more conventional therapeutic agents such as, for example, alkylating agents, folic acid antagonists, anti-metabolites of nucleic acid metabolism, antibiotics, pyrimidine analogs, 5-fluorouracil, purine nucleosides, amines, amino acids, triazol nucleosides, or corticosteroids. Such other agents may be present in the composition being administered or may be administered separately. Also, the antagonist is suitably administered serially or in combination with radiological treatments, whether involving irradiation or administration of radioactive substances.

Treatment with hVEGF antagonists optimally may be suspended during periods of wound healing or desirable neovascularization.

The following examples are offered by way of illustration only and are not intended to limit the invention in any manner.

### EXAMPLE 1

### Preparation of Anti- hVEGF Monoclonal Antibodies

To obtain hVEGF conjugated to keyhole limpet hemocyanin (KLH) for immunization, recombinant hVEGF (165 amino acids), Leung, et al., Science 246:1306 (1989), was mixed with KLH at a 4:1 ratio in the presence of 0.05% glutaraldehyde and the mixture was incubated at room temperature for 3 hours with gentle stirring. The mixture then was dialyzed against phosphate buffered saline (PBS) at 4° C. overnight.

Balb/c mice were immunized four times every two weeks by intraperitoneal injections with 5 µg of hVEGF conjugated to 20 µg of KLH, and were boosted with the same dose of hVEGF conjugated to KLH four days prior to cell fusion.

Spleen cells from the immunized mice were fused with P3X63Ag8U.1 myeloma cells, Yelton, et al., Curr. Top. Microbiol. Immunol. 81:1 (1978), using 35% polyethylene glycol (PEG) as described. Yarmush, et al., Proc. Nat. Acad. Sci. 77:2899 (1980). Hybridomas were selected in HAT medium.

Supernatants from hybridoma cell cultures were screened for anti-hVEGF antibody production by an ELISA assay using hVEGF-coated microtiter plates. Antibody that was bound to hVEGF in each of the wells was determined using alkaline phosphatase-conjugated goat anti-mouse IgG immunoglobulin and the chromogenic substrate p-nitrophenyl phosphate. Harlow & Lane, Antibodies: A Laboratory Manual, p.597 (Cold Spring Harbor Laboratory, 1988). Hybridoma cells thus determined to produce anti-hVEGF antibodies were subcloned by limiting dilution, and two of those clones, designated A4.6.1 and B2.6.2, were chosen for further studies.

### EXAMPLE 2

### Characterization of Anti-hVEGF Monoclonal Antibodies

### A. Antigen Specificity

The binding specificities of the anti-hVEGF monoclonal antibodies produced by the A4.6.1 and B2.6.2 hybridomas were determined by ELISA. The monoclonal antibodies were added to the wells of microtiter plates that previously had been coated with hVEGF, FGF, HGF, or epidermal growth factor (EGF). Bound antibody was detected with peroxidase conjugated goat anti-mouse lgG immunoglobulins. The results of those assays confirmed that the monoclonal antibodies produced by the A4.6.1 and B2.6.2 hybridomas bind to hVEGF, but not detectably to those other protein growth factors.

### B. Epitope Mapping

A competitive binding ELISA was used to determine whether the monoclonal antibodies produced by the A4.6.1 and B2.6.2 hybridomas bind to the same or different epitopes (sites) within hVEGF. Kim, et al., Infect. Immun. 57:944 (1989). Individual unlabeled anti-hVEGF monoclonal antibodies (A4.6.1 or B2.6.2) or irrelevant anti-HGF antibody (IgG1 isotype) were added to the wells of microtiter plates that previously had been coated with hVEGF. Biotinylated anti-hVEGF monoclonal antibodies (BIO-A4.6.1 or BIO-B2.6.2) were then added. The ratio of biotinylated antibody to unlabeled antibody was 1:1000. Binding of the biotinylated antibodies was visualized by the addition of avidin-conjugated peroxidase, followed by o-phenylenediamine dihydrochloride and hydrogen peroxide. The color reaction, indicating the amount of biotinylated antibody bound, was determined by measuring the optical density (O.D) at 495 nm wavelength.

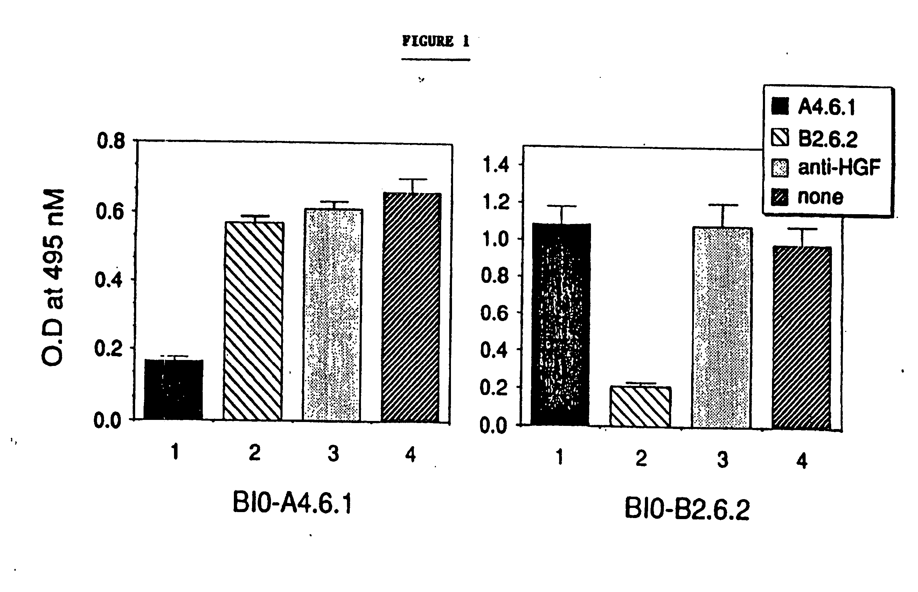

As shown in Figure 1, in each case, the binding of the biotinylated anti-hVEGF antibody was inhibited by the corresponding unlabeled antibody, but not by the other unlabeled anti-hVEGF antibody or the anti-HGF antibody. These results indicate that the monoclonal antibodies produced by the A4.6.1 and B2.6.2 hybridomas bind to different epitopes within hVEGF.

### C. Isotyping

The isotypes of the anti-hVEGF monoclonal antibodies produced by the A4.6.1 and B2.6.2 hybridomas were determined by ELISA. Samples of culture medium (supernatant) in which each of the hybridomas was growing were added to the wells of microtiter plates that had previously been coated with hVEGF. The captured anti-hVEGF monoclonal antibodies were incubated with different isotype-specific alkaline phosphatase-conjugated goat anti-mouse immunoglobulins, and the binding of the conjugated antibodies to the anti-hVEGF monoclonal antibodies was determined by the addition of p-nitrophenyl phosphate. The color reaction was measured at 405 nm with an ELISA plate reader.

By that method, the isotype of the monoclonal antibodies produced by both the A4.6.1 and B2.6.2 hybridomas was determined to be IgG1.

### D. Binding Affinity

The affinities of the anti-hVEGF monoclonal antibodies produced by the A4.6.1 and B2.6.2 hybridomas for hVEGF were determined by a competitive binding assays. A predetermined sub-optimal concentration of monoclonal antibody was added to samples containing 20,000 - 40,000 cpm ¹²⁶I-hVEGF (1 - 2 ng) and various known amounts of unlabeled hVEGF (1 - 1000 ng). After 1 hour at room temperature, 100 µl of goat anti-mouse Ig antisera (Pel-Freez, Rogers, AR USA) were added, and the mixtures were incubated another hour at room temperature. Complexes of antibody and bound protein (immune complexes) were precipitated by the addition of 500 µl of 6% polyethylene glycol (PEG, mol. wt. 8000) at 4° C., followed by centrifugation at 2000 x G. for 20 min. at 4° C. The amount of ¹²⁵I-hVEGF bound to the anti-hVEGF monoclonal antibody in each sample was determined by counting the pelleted material in a gamma counter.

Affinity constants were calculated from the data by Scatchard analysis. The affinity of the anti-hVEGF monoclonal antibody produced by the A4.6.1 hybridoma was calculated to be 1.2 x 10⁹ liters/mole. The affinity of the anti-hVEGF monoclonal antibody produced by the B2.6.2 hybridoma was calculated to be 2.5 x 10⁹ liters/mole.

### E. Inhibition of hVEGF Mitogenic Activity

Bovine adrenal cortex capillary endothelial (ACE) cells, Ferrara, et al., Proc. Nat. Acad. Sci. 84:5773 (1987), were seeded at a density of 10⁴ cells/ml in 12 multiwell plates, and 2.5 ng/ml hVEGF was added to each well in the presence or absence of various concentrations of the anti-hVEGF monoclonal antibodies produced by the A4.6.1 or B2.6.2 hybridomas, or an irrelevant anti-HGF monoclonal antibody. After culturing 5 days, the cells in each well were counted in a Coulter counter. As a control, ACE cells were cultured in the absence of added hVEGF.

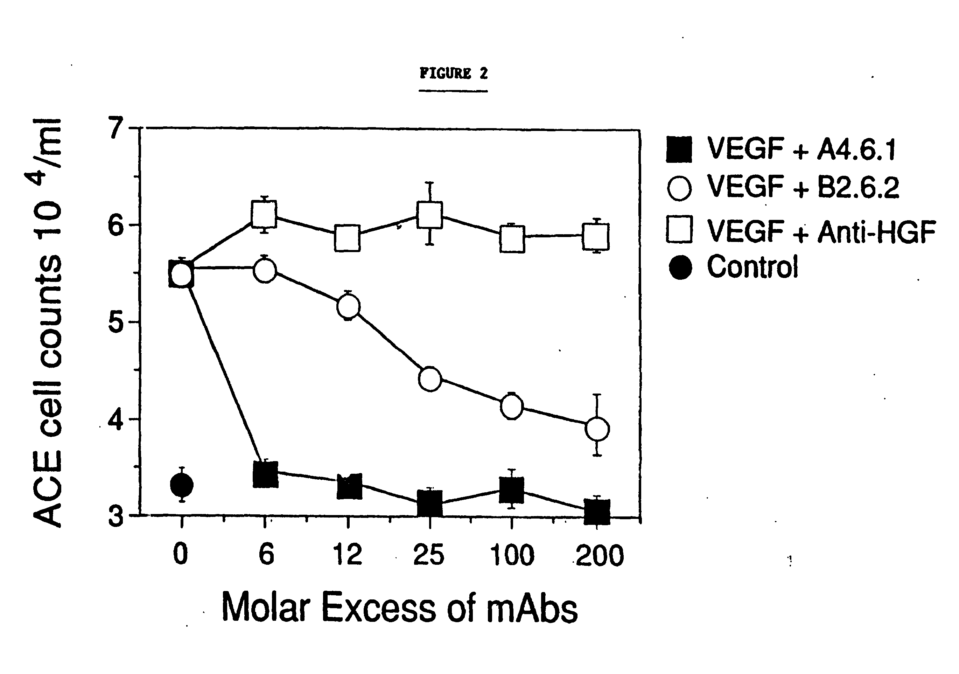

As shown in Figure 2, both of the anti-hVEGF monoclonal antibodies inhibited the ability of the added hVEGF to support the growth or survival of the bovine ACE cells. The monoclonal antibody produced by the A4.6.1 hybridoma completely inhibited the mitogenic activity of hVEGF (greater than about 90% inhibition), whereas the monoclonal antibody produced by the B2.6.2 hybridoma only partially inhibited the mitogenic activity of hVEGF.

### F. Inhibition of hVEGF Binding

Bovine ACE cells were seeded at a density of 2.5 x 10⁴ cells/0.5 ml/well in 24 well microtiter plates in Dulbecco's Modified Eagle's Medium (DMEM) containing 10% calf serum, 2 mM glutamine, and 1 ng/ml basic fibroblast growth factor. After culturing overnight, the cells were washed once in binding buffer (equal volumes of DMEM and F12 medium plus 25 mM HEPES and 1% bovine serum albumin) at 4° C.

12,000 cpm ¹²⁶I-hVEGF (approx. 5 x 10⁴ cpm/ng/ml) was preincubated for 30 minutes with 5 µg of the anti-hVEGF monoclonal antibody produced by the A4.6.1, B2.6.2, or A2.6.1 hybridoma (250 µl total volume), and thereafter the mixtures were added to the bovine ACE cells in the microtiter plates. After incubating the cells for 3 hours at 4° C., the cells were washed 3 times with binding buffer at 4° C., solubilized by the addition of 0.5 ml 0.2 N. NaOH, and counted in a gamma counter.

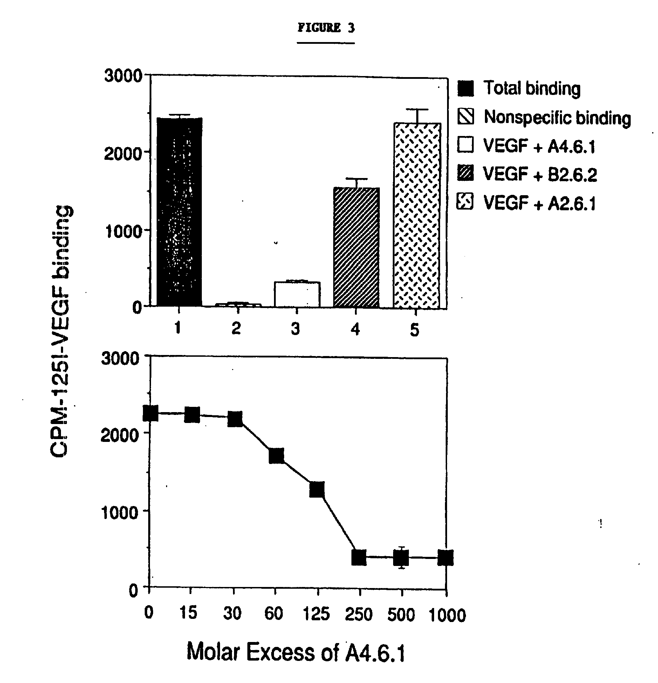

As shown in Figure 3 (upper), the anti-hVEGF monoclonal antibodies produced by the A4.6.1 and B2.6.2 hybridomas inhibited the binding of hVEGF to the bovine ACE cells. In contrast, the anti-hVEGF monoclonal antibody produced by the A2.6.1 hybridoma had no apparent effect on the binding of hVEGF to the bovine ACE cells. Consistent with the results obtained in the cell proliferation assay described above, the monoclonal antibody produced by the A4.6.1 hybridoma inhibited the binding of hVEGF to a greater extent than the monoclonal antibody produced by the B2.6.2 hybridoma.

As shown in Figure 3 (lower), the monoclonal antibody produced by the A4.6.1 hybridoma completely inhibited the binding of hVEGF to the bovine ACE cells at a 1:250 molar ratio of hVEGF to antibody.

### G. Cross-reactivity with other VEGF isoforms

To determine whether the anti-hVEGF monoclonal antibody produced by the A4.6.1 hybridoma is reactive with the 121- and 189-amino acid forms of hVEGF, the antibody was assayed for its ability to immunoprecipate those polypeptides.

Human 293 cells were transfected with vectors comprising the nucleotide coding sequence of the 121- and 189-amino acid hVEGF polypeptides, as described. Leung, et al., Science 246:1306 (1989). Two days after transfection, the cells were transferred to medium lacking cysteine and methionine. The cells were incubated 30 minutes in that medium, then 100 µCi/ml of each ³⁶S-methionine and ³⁶S-cysteine were added to the medium, and the cells were incubated another two hours. The labeling was chased by transferring the cells to serum free medium and incubating three hours. The cell culture media were collected, and the cells were lysed by incubating for 30 minutes in lysis buffer (150 mM NaCI, 1% NP40, 0.5% deoxycholate, 0.1% sodium dodecyl sulfate (SDS), 50 mM Tris, pH 8.0). Cell debris was removed from the lysates by centrifugation at 200 x G. for 30 minutes.

500 µl samples of cell culture media and cell lysates were incubated with 2 µl of A4.6.1 hybridoma antibody (2.4 mg/ml) for 1 hour at 4° C., and then were incubated with 5 µl of rabbit anti-mouse IgG immunoglobulin for 1 hour at 4° C. Immune complexes of ³⁵S-labeled hVEGF and anti-hVEGF monoclonal antibody were precipitated with protein-A Sepharose (Pharmacia), then subjected to SDS - 12% polyacrylamide gel electrophoresis under reducing conditions. The gel was exposed to x-ray film for analysis of the immunoprecipitated, radiolabeled proteins by autoradiography.

The results of that analysis indicated that the anti-hVEGF monoclonal antibody produced by the A4.6.1 hybridoma was cross-reactive with both the 121- and 189-amino acid forms of hVEGF.

### EXAMPLE 3

### Preparation of hVEGF Receptor - IgG Fusion Protein

The nucleotide and amino acid coding sequences of the fit hVEGF receptor are disclosed in Shibuya, et al., Oncogene 5:519-524 (1990). The coding sequence of the extracellular domain of the flt hVEGF receptor was fused to the coding sequence of human lgG1 heavy chain in a two-step process.

Site-directed mutagenesis was used to introduce a BstBI restriction into DNA encoding fit at a site 5' to the codon for amino acid 759 of flt, and to convert the unique BstEII restriction site in plasmid pBSSK^{.}FC, Bennett, et al., J. Biol. Chem. 266:23060-23067 (1991), to a BstBI site. The modified plasmid was digested with EcoRI and BstBI and the resulting large fragment of plasmid DNA was ligated together with an EcoRI-BstBI fragment of the flt DNA encoding the extracellular domain (amino acids 1-758) of the flt hVEGF receptor.

The resulting construct was digested with Clal and Notl to generate an approximately 3.3 kb fragment, which is then inserted into the multiple cloning site of the mammalian expression vector pHEBO2 (Leung, et al., Neuron 8:1045 (1992) by ligation. The ends of 3.3. kb fragment are modified, for example by the addition of linkers, to obtain insertion of the fragment into the vector in the correct orientation for expression.

Mammalian host cells (for example, CEN4 cells (Leung, et al. supra) are transfected with the pHEBO2 plasmid containing the flt insert by electroporation. Transfected cells are cultured in medium containing about 10% fetal bovine serum, 2 mM glutamine, and antibiotics, and at about 75% confluency are transferred to serum free medium. Medium is conditioned for 3-4 days prior to collection, and the flt-IgG fusion protein is purified from the conditioned medium by chromatography on a protein-A affinity matrix essentially as described in Bennett, et al., J. Biol. Chem. 266:23060-23067 (1991).

### EXAMPLE 4 (BACKGROUND)

### Inhibition of Tumor Growth with hVEGF Antagonists

Various human tumor cell lines growing in culture were assayed for production of hVEGF by ELISA. Ovary, lung, colon, gastric, breast, and brain tumor cell lines were found to produce hVEGF. Three cell lines that produced hVEGF, NEG 55 (also referred to as G55) (human glioma cell line obtained from Dr. M. Westphal, Department of Neurosurgery, University Hospital Eppendor, Hamburg, Germany, also referred to as G55), A-673 (human rhabdomyosarcoma cell line obtained from the American Type Culture Collection (ATCC), Rockville, Maryland USA 20852 as cell line number CRL 1598), and SK-LMS-1 (leiomyosarcoma cell line obtained from the ATCC as cell line number HTB 88), were used for further studies.

Six to ten week old female Beige/nude mice (Charles River Laboratory, Wilmington, Massachusetts USA) were injected subcutaneously with 1 - 5 x 10⁸ tumor cells in 100-200 µl PBS. At various times after tumor growth was established, mice were injected intraperitoneally once or twice per week with various doses of A4.6.1 anti-hVEGF monoclonal antibody, an irrelevant anti-gp 120 monoclonal antibody (5B6), or PBS. Tumor size was measured every week, and at the conclusion of the study the tumors were excised and weighed.

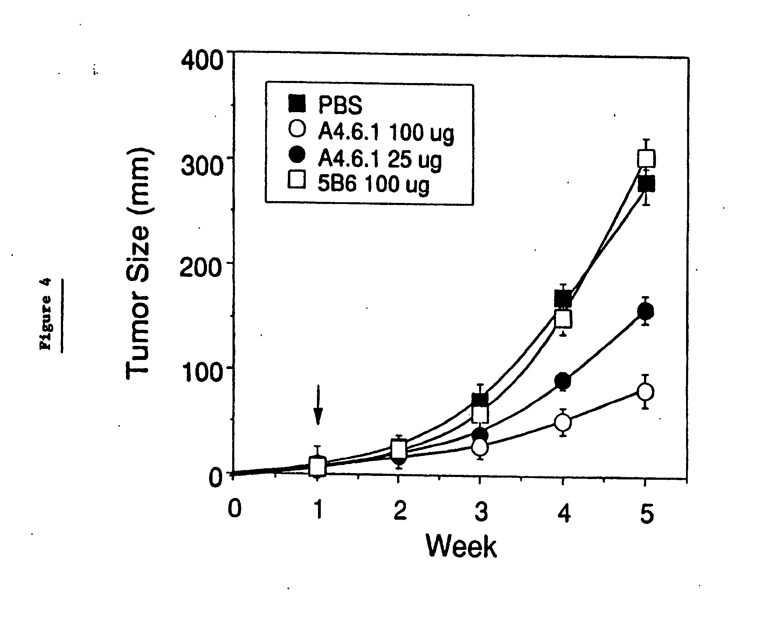

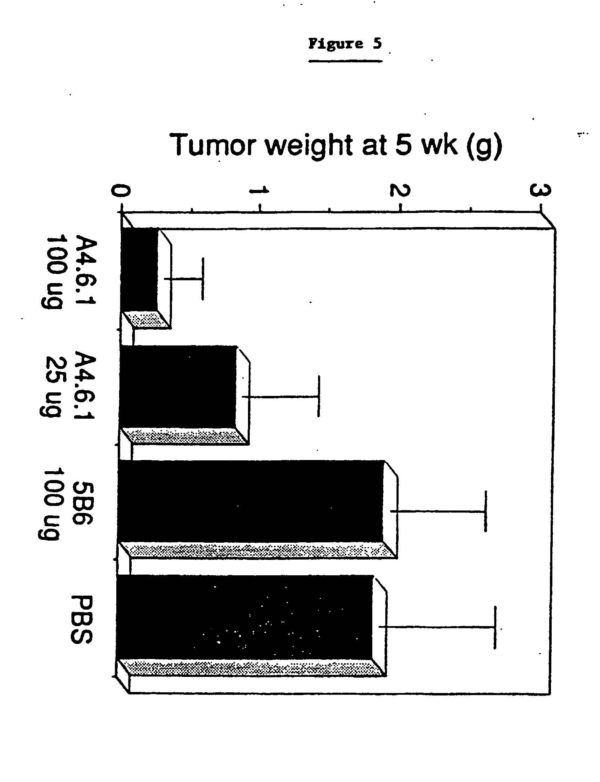

The effect of various amounts of A4.6.1 anti-hVEGF monoclonal antibody on the growth of NEG 55 tumors in mice is shown in Figures 4 and 5. Figure 4 shows that mice treated with 25 µg or 100 µg of A4.6.1 anti-hVEGF monoclonal antibody beginning one week after inoculation of NEG 55 cells had a substantially reduced rate of tumor growth as compared to mice treated with either irrelevant antibody or PBS. Figure 5 shows that five weeks after inoculation of the NEG 55 cells, the size of the tumors in mice treated with A4.6.1 anti-hVEGF antibody was about 50% (in the case of mice treated with 25 µg dosages of the antibody) to 85% (in the case of mice treated with 100 µg dosages of the antibody) less than the size of tumors in mice treated with irrelevant antibody or PBS.

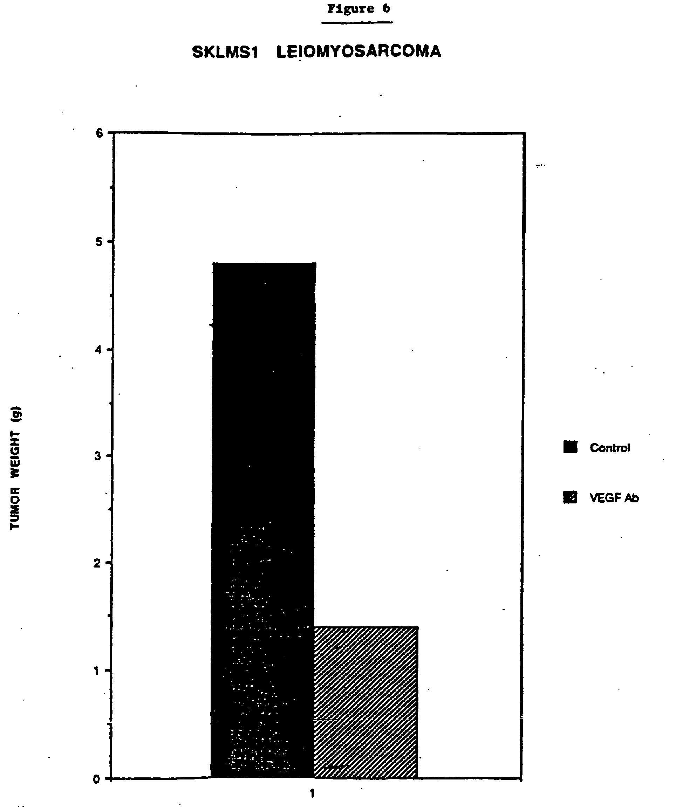

The effect of A4.6.1 anti-hVEGF monoclonal antibody treatment on the growth of SK-LMS-1 tumors in mice is shown in Figure 6. Five weeks after innoculation of the SK-LMS-1 cells, the average size of tumors in mice treated with the A4.6.1 anti-hVEGF antibody was about 75% less than the size tumors in mice treated with irrelevant antibody or PBS.

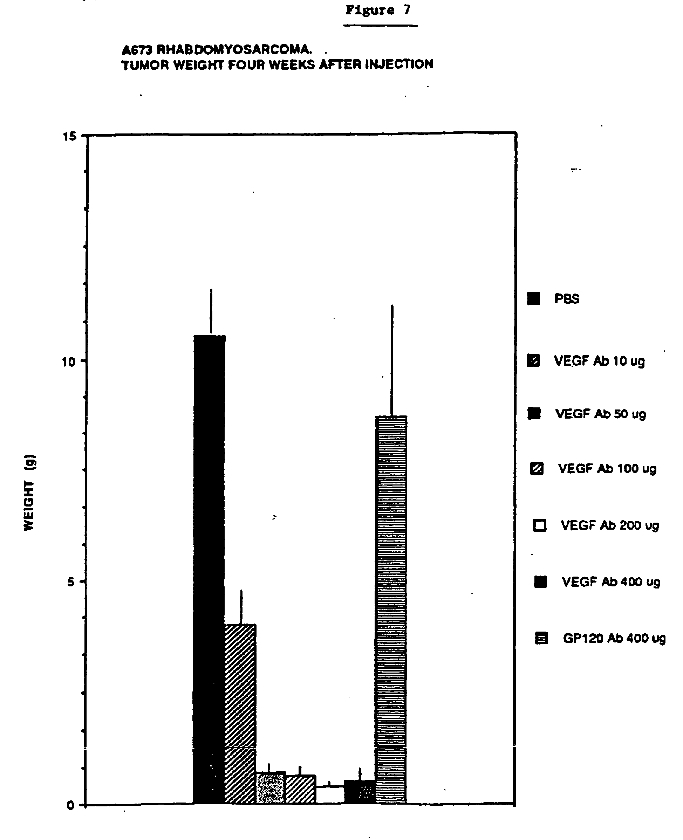

The effect of A4.6.1 anti-hVEGF monoclonal antibody treatment on the growth of A673 tumors in mice is shown in Figure 7. Four weeks after innoculation of the A673 cells, the average size of tumors in mice treated with A4.6.1 anti-hVEGF antibody was about 60% (in the case of mice treated with 10 µg dosages of the antibody) to greater than 90% (in the case of mice treated with 50-400 µg dosages of the antibody) less than the size of tumors in mice treated with irrelevant antibody or PBS.

### EXAMPLE 5

### Analysis of the Direct Effect of Anti-hVEGF Antibody on Tumor Cells Growing in Culture

NEG55 human glioblastoma cells or A673 rhabdomyosarcoma cells were seeded at a density of 7 x 10³ cells/well in multiwells plates (12 wells/plate) in F12/DMEM medium containing 10% fetal calf serum, 2mM glutamine, and antibiotics. A4.6.1 anti-hVEGF antibody then was added to the cell cultures to a final concentration of 0 - 20.0 µg antibody/ml. After five days, the cells growing in the wells were dissociated by exposure to trypsin and counted in a Coulter counter.

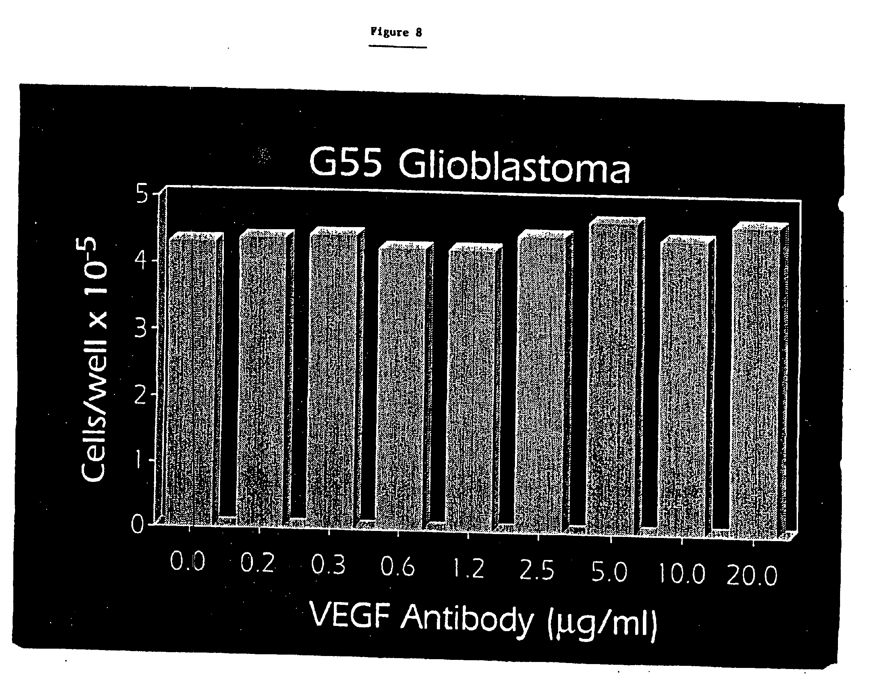

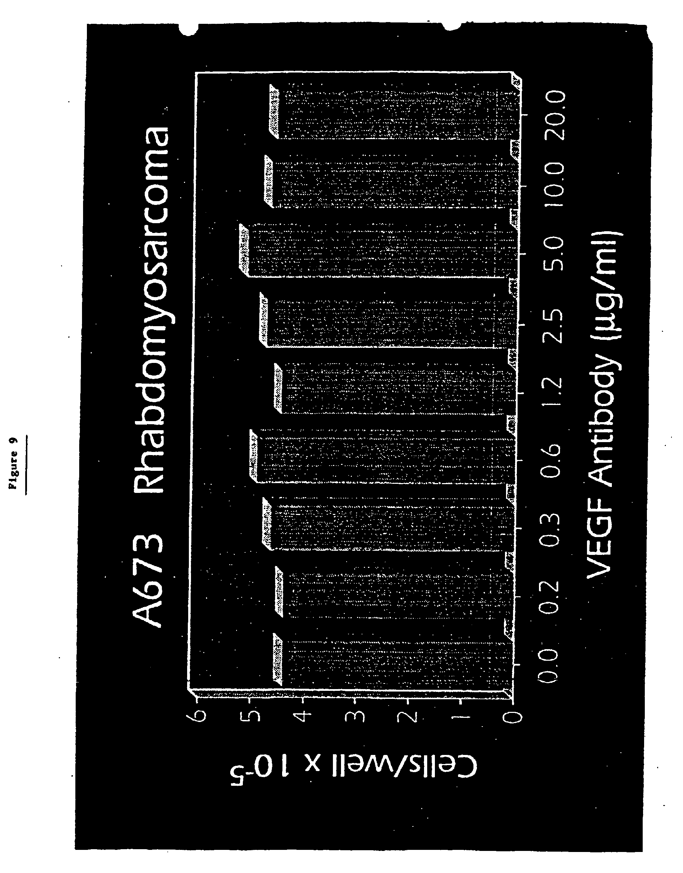

Figures 8 and 9 show the results of those studies. As is apparent, the A4.6.1 anti-hVEGF antibody did not have any significant effect on the growth of the NEG55 or A673 cells in culture. These results indicate that the A4.6.1 anti-hVEGF antibody is not cytotoxic, and strongly suggest that the observed anti-tumor effects of the antibody are due to its inhibition of VEGF-mediated neovascularization.

### EXAMPLE 6

### Effect of Anti-hVEGF Antibody on Endothelial Cell Chemotaxis

Chemotaxis of endothelial cells and others cells, including monocytes and lymphocytes, play an important role in the pathogenesis of rheumatoid arthritis. Endothelial cell migration and proliferation accompany the angiogenesis that occurs in the rheumatoid synovium. Vascularized tissue (pannus) invades and destroys the articular cartilage.

To determine whether hVEGF antagonists interfere with this process, we assayed the effect of the A4.6.1 anti-hVEGF antibody on endothelial cell chemotaxis stimulated by synovial fluid from patients having rheumatoid arthritis. As a control, we also assayed the effect of the A4.6.1 anti-hVEGF antibody on endothelial cell chemotaxis stimulated by synovial fluid from patients having osteoarthritis (the angiogenesis that occurs in rheumatoid arthritis does not occur in osteoarthritis).

Endothelial cell chemotaxis was assayed using modified Boyden chambers according to established procedures. Thompson, et al., Cancer Res. 51:2670 (1991); Phillips, et al., Proc. Exp. Biol. Med. 197:458 (1991). About 10⁴ human umbilical vein endothelial cells were allowed to adhere to gelatin-coated filters (0.8 micron pore size) in 48-well multiwell microchambers in culture medium containing 0.1% fetal bovine serum. After about two hours, the chambers were inverted and test samples (rheumatoid arthritis synovial fluid, osteoarthritis synovial fluid, basic FGF (bFGF) (to a final concentration of 1 µg/ml), or PBS) and A4.6.1 anti-hVEGF antibody (to a final concentration of 10 µg/ml) were added to the wells. After two to four hours, cells that had migrated were stained and counted.

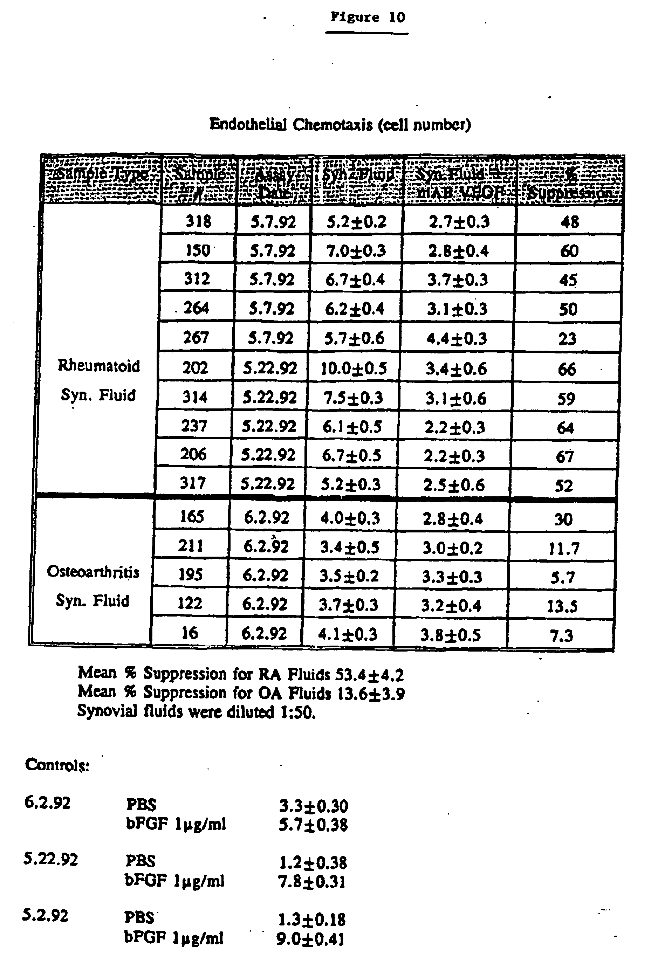

Figure 10 shows the averaged results of those studies. The values shown in the column labeled "Syn. Fluid" and shown at the bottom of the page for the controls are the average number of endothelial cells that migrated in the presence of synovial fluid, bFGF, or PBS alone. The values in the column labeled "Syn. Fluid + mAB VEGF" are the average number of endothelial cells that migrated in the presence of synovial fluid plus added A4.6.1 anti-hVEGF antibody. The values in the column labeled "% Suppression" indicate the percentage reduction in synovial fluid-induced endothelial cell migration resulting from the addition of anti-hVEGF antibody. As indicated, the anti-hVEGF antibody significantly inhibited the ability of rheumatoid arthritis synovial fluid (53.40 average percentage inhibition), but not osteorthritis synovial fluid (13.64 average percentage inhibition), to induce endothelial cell migration.

## Claims

1. Use of a hVEGF antagonist in the preparation of a medicament for the treatment of a non-neoplastic disease or disorder **characterised by** undesirable excessive neovascularisation, wherein the hVEGF antagonist is:
(a) an anti-VEGF antibody or antibody fragment;
(b) an anti-VEGF receptor antibody or antibody fragment; or
(c) an isolated hVEGF receptor.

2. The use according to claim 1 wherein the disease or disorder is rheumatoid arthritis, psoriasis, atherosclerosis, diabetic or other retinopathy, retrolental fibroplasia, neovascular glaucoma, thyroid hyperplasia (including Grave's disease), corneal or other tissue transplantation, or chronic inflammation.

3. The use according to claim 2 wherein the disease or disorder is rheumatoid arthritis.

4. The use according to any preceding claim wherein the anti-VEGF antibody is an anti-hVEGF antibody.

5. The use according to any preceding claim wherein the antibody is a monoclonal antibody.

6. The use according to any preceding claim wherein the antibody is humanised.

7. The use according to any one of claims 1 to 3 wherein the isolated hVEGF receptor consists of the extracellular domain of a hVEGF receptor.

8. The use according to any one of claims 1 to 3 and 7, wherein the hVEGF receptor is the fit receptor.

9. The use according to any one of claims 1 to 3 and 7, wherein the hVEGF receptor is the KDR receptor.

10. The use according to any one of claims 1 to 3 and 7 to 9, wherein the hVEGF receptor is fused to a carrier polypeptide.

11. The use according to claim 10, wherein the carrier polypeptide is an immunoglobulin polypeptide.

12. The use according to claim 11, wherein the immunoglobulin is IgG.

13. The use according to claim 12, wherein the hVEGF antagonist is a flt-lgG fusion protein.

14. An hVEGF antagonist for use in the treatment of a non-neoplastic disease or disorder **characterised by** undesirable excessive neovascularisation, wherein the hVEGF antagonist is:
(a) an anti-VEGF antibody or antibody fragment;
(b) an anti-VEGF receptor antibody or antibody fragment; or
(c) an isolated hVEGF receptor.

15. The hVEGF antagonist according to claim 14 wherein the disease or disorder is rheumatoid arthritis, psoriasis, atherosclerosis, diabetic or other retinopathy, retrolental fibroplasia, neovascular glaucoma, thyroid hyperplasia (including Grave's disease), corneal or other tissue transplantation, or chronic inflammation.

16. The hVEGF antagonist according to claim 15 wherein the disease or disorder is rheumatoid arthritis.

17. The hVEGF antagonist according to any one of claims 14 to 16 wherein the anti-VEGF antibody is an anti-hVEGF antibody.

18. The hVEGF antagonist according to any one of claims 14 to 17 wherein the antibody is a monoclonal antibody.

19. The hVEGF antagonist according to any one of claims 14 to 18 wherein the antibody is humanised.

20. The hVEGF antagonist according to any one of claims 14 to 16 wherein the isolated hVEGF receptor consists of the extracellular domain of a hVEGF receptor.

21. The hVEGF antagonist according to any one of claims 14 to 16 and 20, wherein the hVEGF receptor is the fit receptor.

22. The hVEGF antagonist according to any one of claims 14 to 16 and 20, wherein the hVEGF receptor is the KDR receptor.

23. The hVEGF antagonist according to any one of claims 14 to 16 and 20 to 22, wherein the hVEGF receptor is fused to a carrier polypeptide.

24. The hVEGF antagonist according to claim 23, wherein the carrier polypeptide is an immunoglobulin polypeptide.

25. The hVEGF antagonist according to claim 24, wherein the immunoglobulin is IgG.

26. The hVEGF antagonist according to claim 25, wherein the hVEGF antagonist is a flt-IgG fusion protein.

## Patentansprüche

1. Verwendung eines hVEGF-Antagonisten zur Herstellung eines Medikaments zur Behandlung einer nicht-neoplastischen Erkrankung oder Störung, die durch unerwünschte übermäßige Gefäßneubildung **gekennzeichnet** ist, worin der hVEGF-Antagonist:
(a) ein Anti-VEGF-Antikörper oder -Antikörper-Fragment;
(b) ein Anti-VEGF-Rezeptor-Antikörper oder -Antikörper-Fragment; oder
(c) ein isolierter hVEGF-Rezeptor ist.

2. Verwendung nach Anspruch 1, worin es sich bei der Erkrankung oder Störung um rheumatoide Arthritis, Psoriasis, Atherosklerose, diabetische oder andere Retinopathien, retrolentale Fibroplasie, neovaskuläres Glaukom, Schilddrüsenhyperplasie (umfassend die Basedow-Krankheit), Hornhaut- oder andere Gewebetransplantationen oder chronische Entzündungen handelt.

3. Verwendung nach Anspruch 2, worin es sich bei der Erkrankung oder Störung um rheumatoide Arthritis handelt.

4. Verwendung nach einem der vorangegangenen Ansprüche, worin es sich bei dem Anti-VEGF-Antikörper um einen Anti-hVEGF-Antikörper handelt.

5. Verwendung nach einem der vorangegangenen Ansprüche, worin es sich bei dem Antikörper um einen monoklonalen Antikörper handelt.

6. Verwendung nach einem der vorangegangenen Ansprüche, worin der Antikörper humanisiert ist.

7. Verwendung nach einem der Ansprüche 1 bis 3, worin der isolierte hVEGF-Rezeptor aus der extrazellulären Domäne eines hVEGF-Rezeptors besteht.

8. Verwendung nach einem der Ansprüche 1 bis 3 und 7, worin es sich bei dem hVEGF-Rezeptor um den flt-Rezeptor handelt.

9. Verwendung nach einem der Ansprüche 1 bis 3 und 7, worin es sich bei dem hVEGF-Rezeptor um den KDR-Rezeptor handelt.

10. Verwendung nach einem der Ansprüche 1 bis 3 und 7 bis 9, worin der hVEGF-Rezeptor an ein Träger-Polypeptid fusioniert ist.

11. Verwendung nach Anspruch 10, worin es sich bei dem Träger-Polypeptid um ein Immunglobulin-Polypeptid handelt.

12. Verwendung nach Anspruch 11, worin es sich bei dem Immunglobulin um IgG handelt.

13. Verwendung nach Anspruch 12, worin es sich bei dem hVEGF-Antagonisten um ein flt-IgG-Fusionsprotein handelt.

14. hVEGF-Antagonist zur Verwendung in der Behandlung einer nicht-neoplastischen Erkrankung oder Störung, die durch unerwünschte übermäßige Gefäßneubildung **gekennzeichnet** ist, worin der hVEGF-Antagonist:
(a) ein Anti-VEGF-Antikörper oder -Antikörper-Fragment;
(b) ein Anti-VEGF-Rezeptor-Antikörper oder -Antikörper-Fragment; oder
(c) ein isolierter hVEGF-Rezeptor ist.

15. hVEGF-Antagonist nach Anspruch 14, worin es sich bei der Erkrankung oder Störung um rheumatoide Arthritis, Psoriasis, Atherosklerose, diabetische oder andere Retinopathien, retrolentale Fibroplasie, neovaskuläres Glaukom, Schilddrüsenhyperplasie (umfassend die Basedow-Krankheit), Hornhaut- oder andere Gewebetransplantationen oder chronische Entzündungen handelt.

16. hVEGF-Antagonist nach Anspruch 15, worin es sich bei der Erkrankung oder Störung um rheumatoide Arthritis handelt.

17. hVEGF-Antagonist nach einem der Ansprüche 14 bis 16, worin es sich bei dem Anti-VEGF-Antikörper um einen Anti-hVEGF-Antikörper handelt.

18. hVEGF-Antagonist nach einem der Ansprüche 14 bis 17, worin es sich bei dem Antikörper um einen monoklonalen Antikörper handelt.

19. hVEGF-Antagonist nach einem der Ansprüche 14 bis 18, worin der Antikörper humanisiert ist.

20. hVEGF-Antagonist nach einem der Ansprüche 14 bis 16, worin der isolierte hVEGF-Rezeptor aus der extrazellulären Domäne eines hVEGF-Rezeptors besteht.

21. hVEGF-Antagonist nach einem der Ansprüche 14 bis 16 und 20, worin es sich bei dem hVEGF-Rezeptor um den fit-Rezeptor handelt.

22. hVEGF-Antagonist nach einem der Ansprüche 14 bis 16 und 20, worin es sich bei dem hVEGF-Rezeptor um den KDR-Rezeptor handelt.

23. hVEGF-Antagonist nach einem der Ansprüche 14 bis 16 und 20 bis 22, worin der hVEGF-Rezeptor an ein Träger-Polypeptid fusioniert ist.

24. hVEGF-Antagonist nach Anspruch 23, worin es sich bei dem Träger-Polypeptid um ein Immunglobulin-Polypeptid handelt.

25. hVEGF-Antagonist nach Anspruch 24, worin es sich bei dem Immunglobulin um IgG handelt.

26. hVEGF-Antagonist nach Anspruch 25, worin es sich bei dem hVEGF-Antagonisten um ein flt-lgG-Fusionsprotein handelt.

## Revendications

1. Utilisation d'un antagoniste du hVEGF dans la préparation d'un médicament destiné au traitement d'une maladie ou d'un trouble non néoplasique **caractérisé par** une néovascularisation excessive indésirable, dans laquelle l'antagoniste du hVEGF est :
(a) un anticorps ou fragment d'anticorps anti-VEGF ;
(b) un anticorps ou fragment d'anticorps anti-récepteur de VEGF ; ou
(c) un récepteur de hVEGF isolé.

2. Utilisation suivant la revendication 1, dans laquelle la maladie ou le trouble est la polyarthrite rhumatoïde, le psoriasis, l'athérosclérose, la rétinopathie diabétique ou une autre rétinopathie, la fibroplasie rétrolentale, le glaucome néovasculaire, l'hyperplasie thyroïdienne (y compris la maladie de Graves), une transplantation de cornée ou d'un autre tissu ou une inflammation chronique.

3. Utilisation suivant la revendication 2, dans laquelle la maladie ou le trouble est la polyarthrite rhumatoïde.

4. Utilisation suivant l'une quelconque des revendications précédentes, dans, laquelle l'anticorps anti-VEGF est un anticorps anti-hVEGF.

5. Utilisation suivant l'une quelconque des revendications précédentes, dans laquelle l'anticorps est un anticorps monoclonal.

6. Utilisation suivant l'une quelconque des revendications précédentes, dans laquelle l'anticorps est humanisé.

7. Utilisation suivant l'une quelconque des revendications 1 à 3, dans laquelle le récepteur de hVEGF isolé consiste en le domaine extracellulaire d'un récepteur de hVEGF.

8. Utilisation suivant l'une quelconque des revendications 1 à 3 et 7, dans laquelle le récepteur de hVEGF est le récepteur flt.

9. Utilisation suivant l'une quelconque des revendications 1 à 3 et 7, dans laquelle le récepteur de hVEGF est le récepteur KDR.

10. Utilisation suivant l'une quelconque des revendications 1 à 3 et 7 à 9, dans laquelle le récepteur de hVEGF est fusionné à un polypeptide de support.

11. Utilisation suivant la revendication 10, dans laquelle le polypeptide de support est un polypeptide du type immunoglobuline.

12. Utilisation suivant la revendication 11, dans laquelle l'immunoglobuline est une IgG.

13. Utilisation suivant la revendication 12, dans laquelle l'antagoniste du hVEGF est une protéine de fusion flt-IgG.

14. Antagoniste du hVEGF destiné à être utilisé dans le traitement d'une maladie ou d'un trouble non néoplasique **caractérisé par** une néovascularisation excessive indésirable, l'antagoniste du hVEGF étant :
(a) un anticorps ou fragment d'anticorps anti-VEGF ;
(b) un anticorps ou fragment d'anticorps anti-récepteur de VEGF ; ou
(c) un récepteur de hVEGF isolé.

15. Antagoniste du hVEGF suivant la revendication 14, dans lequel la maladie ou le trouble est la polyarthrite rhumatoïde, le psoriasis, l'athérosclérose, la rétinopathie diabétique ou une autre rétinopathie, la fibroplasie rétrolentale, le glaucome néovasculaire, l'hyperplasie thyroïdienne (y compris la maladie de Graves), une transplantation de la cornée ou d'un autre tissu ou une inflammation chronique.

16. Antagoniste du hVEGF suivant la revendication 15, dans lequel la maladie ou le trouble est la polyarthrite rhumatoïde.

17. Antagoniste du hVEGF suivant l'une quelconque des revendications 14 à 16, dans lequel l'anticorps anti-VEGF est un anticorps anti-hVEGF.

18. Antagoniste du hVEGF suivant l'une quelconque des revendications 14 à 17, dans lequel l'anticorps est un anticorps monoclonal.

19. Antagoniste du hVEGF suivant l'une quelconque des revendications 14 à 18, dans lequel l'anticorps est humanisé.

20. Antagoniste du hVEGF suivant l'une quelconque des revendications 14 à 16, dans lequel le récepteur du hVEGF isolé consiste en le domaine extracellulaire d'un récepteur de hVEGF.

21. Antagoniste du hVEGF suivant l'une quelconque des revendications 14 à 16 et 20, dans lequel le récepteur du hVEGF est le récepteur flt.

22. Antagoniste du hVEGF suivant l'une quelconque des revendications 14 à 16 et 20, dans lequel le récepteur du hVEGF est le récepteur KDR.

23. Antagoniste du hVEGF suivant l'une quelconque des revendications 14 à 16 et 20 à 22, dans lequel le récepteur de hVEGF est fusionné à un polypeptide de support.

24. Antagoniste du hVEGF suivant la revendication 23, dans lequel le polypeptide de support est un polypeptide du type immunoglobuline.

25. Antagoniste du hVEGF suivant la revendication 24, dans lequel l'immunoglobuline est une IgG.

26. Antagoniste du hVEGF suivant la revendication 25, ledit antagoniste du hVEGF étant une protéine de fusion flt-IgG.
